# EUROPEAN PATENT APPLICATION

(11) **EP 4 711 038 A1**
(43) Date of publication of application: **18.03.2026**
(21) Application number: 25200535.0
(22) Date of filing: 05.09.2025
(51) Int. Cl.: B01L 1/00, B01L 3/00, B01L 5/02

(54) **FLUIDIC SYSTEM, CONTROL METHOD, SEQUENCING SYSTEM, AND STORAGE MEDIUM**

(30) Priority: 12.09.2024 CN 202411284536
(71) Applicant: GeneMind Biosciences Company Limited, Shenzhen City, Guangdong 518023 (CN)
(72) Inventor: ZHAO, Lifeng, Shenzhen City, 518023 (CN); WU, Ping, Shenzhen City, 518023 (CN); HAN, Weichun, Shenzhen City, 518023 (CN); SONG, Qi, Shenzhen City, 518023 (CN)
(74) Representative: Schiweck Weinzierl Koch Patentanwälte Partnerschaft mbB

(57) **Abstract**

The present application discloses a fluidic system, a control method, a sequencing system, and a storage medium. The fluidic system includes a reaction device, a pipeline, and a degassing device. The reaction device is configured to provide a site for carrying out biochemical reactions. The pipeline is in communication with the reaction device and configured to deliver liquid to the reaction device and/or receive liquid flowing out from the reaction device. The degassing device is connected to the pipeline and configured to remove gas from the liquid in the pipeline. In this way, by removing gas from the liquid in the pipeline using the degassing device, the formation of bubbles in the reaction device can be reduced.

## Description

### TECHNICAL FIELD

The present disclosure relates to the field of gene sequencing, and in particular, to a fluidic system, a control method, a sequencing system, and a storage medium.

### BACKGROUND

Gene sequencing technology refers to the technical means of acquiring the base sequence of DNA or RNA by assays. The current dominant sequencing technology is high-throughput sequencing. In a sequencing platform that achieves high-throughput sequencing based on sequencing by synthesis, the general gene sequencing process includes: fixing a nucleic acid sample under test on a flow cell, for example, by hybridization; forming a nucleic acid molecule cluster on the nucleic acid sample under test by using PCR amplification; adding sequencing reagents (e.g., bases with fluorophores, polymerases, and primers) to the flow cell through a fluidic system; bonding the bases with the fluorophores to the base on the nucleic acid sample under test according to the base complementary pairing principle; exciting the fluorophores by an optical imaging system to generate fluorescence; collecting the fluorescence for forming an image; and performing base calling on the image, so as to achieve base sequence determination of the nucleic acid sample under test.

However, if gas is present in the sequencing reagents and/or nucleic acid sample under test, the gas will enter the flow cell along with the sequencing reagents and/or nucleic acid sample under test, resulting in the formation of bubbles within the flow cell, thereby affecting image quality and subsequently impacting sequencing quality.

### SUMMARY

The present application, to at least solve one of the technical problems existing in the prior art, provides a fluidic system, a control method, a sequencing system, and a storage medium.

In one aspect, the present application provides a fluidic system. The fluidic system includes a reaction device, a pipeline, and a degassing device. The reaction device is configured to provide a site for carrying out biochemical reactions. The pipeline is in communication with the reaction device and configured to deliver liquid to the reaction device and/or receive liquid flowing out from the reaction device. The degassing device is connected to the pipeline and configured to remove gas from the liquid in the pipeline.

In this way, by removing gas from the liquid in the pipeline using the degassing device, the formation of bubbles by the gas in the reaction device can be reduced or avoided.

In some embodiments, the reaction device includes a liquid inlet and a liquid outlet.

The pipeline includes a first pipeline and a second pipeline, where the first pipeline is in communication with the liquid inlet, and the second pipeline is in communication with the liquid outlet; the first pipeline and/or the second pipeline is connected to the degassing device.

In some embodiments, the reaction device includes a liquid inlet and a liquid outlet; and the pipeline includes a bypass pipeline, where one end of the bypass pipeline is in communication with the liquid inlet, and the other end is in communication with the liquid outlet; the bypass pipeline is connected to the degassing device.

In some embodiments, the degassing device includes a sealed chamber; the pipeline passes through the sealed chamber, and the portion of the pipeline located within the sealed chamber is capable of, when the sealed chamber is under a negative pressure condition, allowing the gas in the liquid in the pipeline to permeate out, so that the liquid in the pipeline is degassed after passing through the sealed chamber.

In some embodiments, the degassing device includes a negative pressure device and an exhaust pipeline, where one end of the exhaust pipeline is in communication with the sealed chamber, and the negative pressure device is arranged on the exhaust pipeline and configured to generate a negative pressure in the sealed chamber.

In some embodiments, the degassing device includes a detector located between the negative pressure device and the sealed chamber, where the detector is in communication with the exhaust pipeline and configured to detect an air pressure in the sealed chamber through the exhaust pipeline.

In some embodiments, the degassing device includes a multi-way valve arranged on the exhaust pipeline and located between the detector and the negative pressure device, where the multi-way valve is capable of enabling the negative pressure device to communicate with either the sealed chamber or the atmosphere.

In another aspect, the present application provides a method for controlling degassing of liquid in a fluidic system. The method includes:
determining whether the degassing device is functioning properly; and
controlling, in the case where the degassing device is functioning properly, the liquid in the pipeline to pass through the degassing device for degassing.

In some embodiments, determining whether the degassing device is functioning properly includes:
acquiring the air pressure in the sealed chamber by using the detector; and
determining whether the degassing device is functioning properly according to changes in the air pressure.

In some embodiments, determining whether the degassing device is functioning properly according to the changes in the air pressure includes:
acquiring a first air pressure in the sealed chamber at an initial moment;
acquiring a second air pressure in the sealed chamber after a preset time interval;
calculating to obtain the change rate of the air pressure by using the first air pressure, the second air pressure, and the preset time interval; and
comparing the change rate of the air pressure with a preset change rate to determine whether the degassing device is functioning properly.

In some embodiments, comparing the air pressure change rate with the preset change rate to determine whether the degassing device is functioning properly includes:
when the negative pressure device is operating, determining, if the change rate of the air pressure is greater than or equal to the preset change rate, that the degassing device is functioning properly; or determining, if the change rate of the air pressure is less than the preset change rate, that the degassing device is malfunctioning.

In some embodiments, comparing the air pressure change rate with the preset change rate to determine whether the degassing device is functioning properly includes:
when the negative pressure device stops operating, determining, if the change rate of the air pressure is less than or equal to the preset change rate, that the degassing device is functioning properly; or determining, if the change rate of the air pressure is greater than the preset change rate, that the degassing device is malfunctioning.

In some embodiments, determining whether the degassing device is functioning properly according to the changes in the air pressure includes:
acquiring, when the negative pressure device stops operating, a first moment when the air pressure is at a first threshold value and acquiring a second moment when the air pressure is at a second threshold value, where the second threshold value is greater than the first threshold value;
calculating to obtain a time difference by using the first moment and the second moment; and
comparing the time difference with a preset time difference to determine whether the degassing device is functioning properly.

In some embodiments, comparing the time difference with the preset time difference to determine whether the degassing device is functioning properly includes:
determining, if the time difference is greater than or equal to the preset time difference, that the degassing device is functioning properly; or determining, if the time difference is less than the preset time difference, that the degassing device is malfunctioning.

In some embodiments, determining whether the degassing device is functioning properly according to the changes in the air pressure includes:
recording, when the negative pressure device stops operating, the air pressure as a first threshold value;
starting, when the air pressure is at a second threshold value, the negative pressure device; and
determining, if the air pressure is greater than the first threshold value within a preset time, that the degassing device is malfunctioning; or determining, if the air pressure is less than or equal to the first threshold value within the preset time, that the degassing device is functioning properly, where the second threshold value is greater than the first threshold value.

In some embodiments, determining whether the degassing device is functioning properly according to the changes in the air pressure includes:
recording, when the negative pressure device stops operating, the air pressure as a first threshold value;
determining, at the moment of the preset time interval, whether the air pressure in the sealed chamber is greater than the first threshold value;
acquiring, if the air pressure in the sealed chamber is greater than the first threshold value, a change rate of the air pressure; and
determining, if the change rate of the air pressure is greater than the preset change rate, that the degassing device is malfunctioning; otherwise, determining that the degassing device is functioning properly.

In some embodiments, the method further includes:
starting, if the degassing device is functioning properly, the negative pressure device; or
not starting, if the degassing device is malfunctioning, the negative pressure device.

In some embodiments, the degassing device includes a first degassing device, and the first pipeline is connected to the first degassing device.
controlling, in the case where the degassing device is functioning properly, the liquid in the pipeline to pass through the degassing device for degassing includes:
controlling the liquid in the first pipeline to pass through the first degassing device for degassing, and causing the degassed liquid to enter the reaction device.

In some embodiments, the degassing device includes a second degassing device, and the second pipeline is connected to the second degassing device.

Controlling, in the case where the degassing device is functioning properly, the liquid in the pipeline to pass through the degassing device for degassing includes:
controlling the liquid that flows out of the reaction device and enters the second pipeline to pass through the second degassing device for degassing.

In some embodiments, the degassing device includes a third degassing device.

The pipeline includes a bypass pipeline, where one end of the bypass pipeline is in communication with the liquid inlet, and the other end is in communication with the liquid outlet; the bypass pipeline is connected to the third degassing device.

Controlling, in the case where the degassing device is functioning properly, the liquid in the pipeline to pass through the degassing device for degassing includes:
controlling the liquid that flows out of the reaction device to enter the bypass pipeline, and causing the liquid in the bypass pipeline to enter the third degassing device for degassing.

In some embodiments, the method further includes:
causing the degassed liquid in the bypass pipeline to enter the reaction device.

In yet another aspect, the present application provides a sequencing system. The sequencing system includes the fluidic system described above.

In addition, the present application further provides a computer storage medium. A computer program, when run by a processor, causes the processor to implement the method according to any one of the above embodiments.

Additional aspects and advantages of the present disclosure will in part be illustrated in the following description and become apparent from the following description, or may be learned by the implementation of the present disclosure.

### BRIEF DESCRIPTION OF THE DRAWINGS

The aforementioned and/or additional aspects and advantages of the present disclosure will become apparent and easily understood from the description of the embodiments with reference to the following drawings, in which:
FIG 1 is a schematic structural diagram of a fluidic system according to an embodiment of the present disclosure;
FIG 2 is a schematic structural diagram of a degassing device according to an embodiment of the present disclosure;
FIG 3 is a schematic structural diagram of a degassing device according to an embodiment of the present disclosure;
FIG 4 is a schematic flowchart of a method according to an embodiment of the present disclosure;
FIG 5 is a schematic flowchart of a method according to an embodiment of the present disclosure;
FIG 6 is a schematic flowchart of a method according to an embodiment of the present disclosure; and
FIG 7 is a schematic flowchart of a method according to an embodiment of the present disclosure.

Description of reference numerals: 100, fluidic system; 10, reaction device; 11, liquid inlet; 12, liquid outlet; 20, pipeline; 21, first pipeline; 22, second pipeline; 23, bypass pipeline; 30, degassing device; 31, sealed chamber; 32, negative pressure device; 33, exhaust pipeline; 34, detector; 35, three-way connector; 36, multi-way valve; 37, first degassing device; 38, second degassing device; 39, third degassing device.

### DETAILED DESCRIPTION

The embodiments of the present disclosure are described in detail below, and the examples of the embodiments are shown in the drawings, throughout which identical or similar reference numerals represent identical or similar elements or elements having identical or similar functionality. The embodiments described below with reference to the drawings are exemplary and are merely intended to illustrate the present disclosure, and should not be construed as limiting the present disclosure.

In the description of the present disclosure, it should be understood that orientational or positional relationships indicated by terms such as "central", "longitudinal", "transverse", "length", "width", "thickness", "upper", "lower", "front", "rear", "left", "right", "vertical", "horizontal", "top", "bottom", "inner", "outer", "clockwise", or "counterclockwise", are those shown on the basis of the drawings, and are merely intended to facilitate and simplify the description rather than indicate or imply that the indicated apparatus or element must have a specific orientation and be configured and operated according to the specific orientation. Such relationships should not be construed as limiting the present disclosure. In addition, the terms "first" and "second" are used herein for descriptive purposes only and should not be construed as indicating or implying relative importance or implicitly indicating the number of technical features described. Therefore, features defined with "first" and "second" may explicitly or implicitly include one or more of the features. In the description of the present disclosure, unless otherwise clearly and specifically defined, the term "plurality" means two or more.

In the description of the present disclosure, it should be noted that unless otherwise clearly specified and defined, the terms "mount", "link", and "connect" should be interpreted in their broad sense. For example, the connection may be a fixed connection, detachable connection, or integral connection; a mechanical connection, electric connection, or communicative connection; or a direct connection, indirect connection through an intermediate, internal communication of two elements, or interaction between two elements. For those of ordinary skill in the art, the specific meanings of the aforementioned terms in the present disclosure can be interpreted according to specific conditions.

In the present disclosure, unless otherwise clearly specified and defined, a first feature being "above" or "below" a second feature may include that the first and second features are in direct contact and that the first and second features are not in direct contact but are in contact via an additional feature therebetween. Moreover, a first feature being "on", "over", and "above" a second feature includes that the first feature is right above or obliquely above the second feature, or simply means that the first feature is at a vertically higher position than the second feature. A first feature being "under", "beneath", and "below" a second feature includes that the first feature is right below or obliquely below the second feature, or simply means that the first feature is at a vertically lower position than the second feature.

The following disclosure provides many different embodiments or examples for implementing different structures of the present disclosure. To simplify the disclosure of the present application, the components and settings of specific examples are described below. Certainly, the examples are merely exemplary and are not intended to limit the present disclosure. In addition, reference numerals and/or characters may be repeatedly used in different examples in the present disclosure for simplicity and clarity rather than to indicate the relationship between various embodiments and/or settings discussed. In addition, the present disclosure provides examples of various specific processes and materials, but those of ordinary skill in the art will recognize the application of other processes and/or the use of other materials.

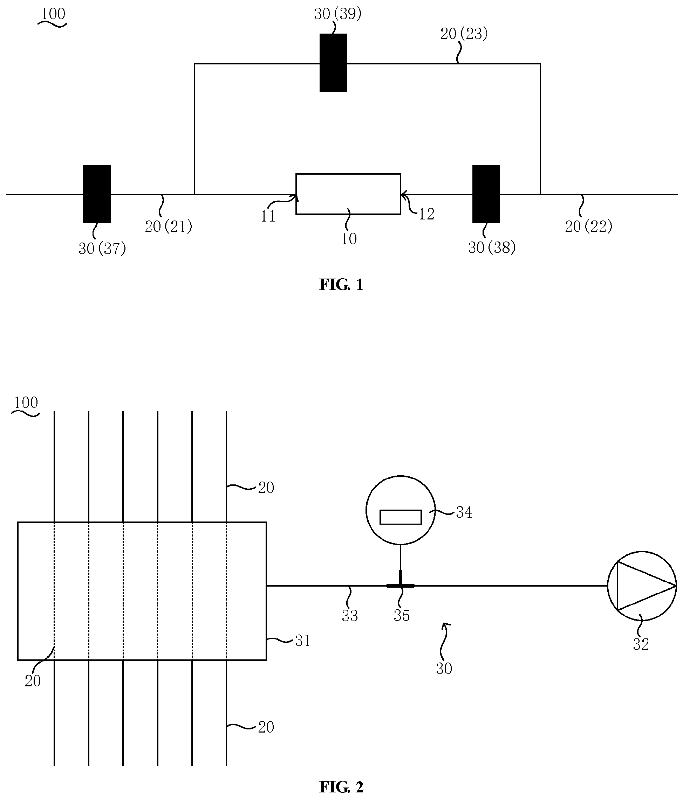

Referring to FIG 1, the present application provides a fluidic system 100. The fluidic system 100 includes a reaction device 10, a pipeline 20, and a degassing device 30. The reaction device 10 is configured to provide a site for carrying out biochemical reactions. The pipeline 20 is in communication with the reaction device 10, and the pipeline 20 is configured to deliver liquid to the reaction device 10 and/or receive liquid flowing out from the reaction device 10. The degassing device 30 is connected to the pipeline 20, and the degassing device 30 is configured to remove gas from the liquid in the pipeline 20.

In this way, by removing gas from the liquid in the pipeline 20 using the degassing device 30, the formation of bubbles in the reaction device 10 can be reduced.

Specifically, the reaction device 10 may be a flow cell, which can also be referred to as a flowcell. The flowcell includes a plurality of fluid channels, each fluid channel being provided with a space for accommodating liquid and being able to accommodate a sample under test and a reagent, so that the sample under test and the reagent undergo a biochemical reaction.

The pipeline 20 may be a conduit for transporting liquid. The cross-sectional shape of the pipeline 20 may be circular, and the length and diameter of the pipeline 20 may be set according to actual needs. The degassing device 30 may be connected to a plurality of pipelines 20 in order to remove gas from the liquid in the plurality of pipelines 20. The gas in the liquid may be gas originally present in the liquid, such as an inhibitory gas added to the reagent to maintain the stability of the reagent. The gas in the liquid may also be gas that is precipitated from the liquid due to excessively high negative pressure in the pipeline 20, or gas dissolved in the liquid.

Referring to FIG 1, in some embodiments, the reaction device 10 includes a liquid inlet 11 and a liquid outlet 12. The pipeline 20 includes a first pipeline 21 and a second pipeline 22. The first pipeline 21 is in communication with the liquid inlet 11, and the second pipeline 22 is in communication with the liquid outlet 12. The first pipeline 21 and/or the second pipeline 22 is connected to the degassing device 30.

Specifically, the first pipeline 21 may be connected to the degassing device 30, or the second pipeline 22 may be connected to the degassing device 30, or both the first pipeline 21 and the second pipeline 22 may be connected to the degassing device 30.

When delivering liquid to the reaction device 10, the liquid may enter the reaction device 10 from the liquid inlet 11 via the first pipeline 21 after being degassed by the degassing device 30, or may enter the reaction device 10 from the liquid outlet 12 via the second pipeline 22 after being degassed by the degassing device 30. In this way, the liquid can be degassed in advance before flowing into the reaction device 10, reducing or avoiding the formation of bubbles by the gas in the reaction device 10.

When receiving liquid flowing out from the reaction device 10, the liquid may flow out of the reaction device 10 from the liquid outlet 12 and be stored in the second pipeline 22 after being degassed by the degassing device 30 in the second pipeline 22. Subsequently, the liquid is degassed by the degassing device 30 in the second pipeline 22 and then enters the reaction device 10 from the liquid outlet 12 for reuse, thereby lowering the cost.

The liquid inlet 11 and the liquid outlet 12 are respectively located at two ends of the fluid channel of the reaction device 10. The number of fluid channels may be plural, with each fluid channel including one liquid inlet 11 and one liquid outlet 12. The number of first pipelines 21 and second pipelines 22 may also be plural, with the plurality of fluid channels being arranged in one-to-one correspondence with the plurality of first pipelines 21 and the plurality of second pipelines 22. Referring to FIG 1, in some embodiments, the pipeline 20 includes a bypass pipeline 23. One end of the bypass pipeline 23 is in communication with the liquid inlet 11, and the other end is in communication with the liquid outlet 12. The bypass pipeline 23 is connected to the degassing device 30.

Specifically, the end of the bypass pipeline 23 that is in communication with the liquid inlet 11 may be located between the liquid inlet 11 and the degassing device 30 of the first pipeline 21. The degassing device 30 of the second pipeline 22 may be located between the end of the bypass pipeline 23 that is in communication with the liquid outlet 12 and the liquid outlet 12.

The liquid flowing out from the reaction device 10 may be degassed by the degassing device 30 in the second pipeline 22 and then stored in the bypass pipeline 23. Then, after being degassed by the degassing device 30 in the bypass pipeline 23, the liquid enters the first pipeline 21 and enters the reaction device 10 from the liquid inlet 11. Alternatively, after being degassed by the degassing device 30 in the second pipeline 22, the liquid enters the bypass pipeline 23, and after being degassed by the degassing device 30 in the bypass pipeline 23, enters the first pipeline 21. After being degassed by the degassing device 30 in the first pipeline 21, the liquid is stored in the first pipeline 21 or a storage container. Then, after being degassed again by the degassing device 30 in the first pipeline 21, the liquid enters the reaction device 10 from the liquid inlet 11. Compared to the case where the liquid flows back from the second pipeline 22 through the reaction device 10 to the first pipeline 21, the liquid flowing back through the bypass pipeline 23 can reduce the flow resistance of the liquid and increase the flow rate of the liquid, thereby improving the recovery efficiency of the liquid.

In the process of liquid recovery and reuse, the liquid may be degassed by a plurality of degassing devices 30, thereby increasing the effective degassing area of the pipeline 20, improving the degassing effect, and at the same time reducing the length or area of the pipeline 20, reducing the use of materials, and further lowering the cost of the fluidic system 100 and the flow resistance of the liquid.

Referring to FIG 2, in some embodiments, the degassing device 30 includes a sealed chamber 31. The pipeline 20 passes through the sealed chamber 31, and a portion of the pipeline 20 located within the sealed chamber 31 is capable of, when the sealed chamber 31 is under a negative pressure condition, allowing the gas in the liquid in the pipeline 20 to permeate out, so that the liquid in the pipeline 20 is degassed after passing through the sealed chamber 31.

Specifically, the sealed chamber 31 may be a sealed space formed from polyvinyl chloride. The sealed chamber 31 may have a shape such as a cuboid or a cylinder. The passage of the pipeline 20 through the sealed chamber 31 may be that the pipeline 20 penetrates the same side wall of the sealed chamber 31, or penetrates two adjacent side walls of the sealed chamber 31, or penetrates two opposite side walls of the sealed chamber 31.

The portion of the pipeline 20 located within the sealed chamber 31 may be a Teflon tube. Teflon tubes have the characteristic of being gas-permeable but water-impermeable, so the gas in the liquid can be discharged from the Teflon tube under the action of negative pressure when passing through the sealed chamber 31, and the discharged gas is located in the sealed chamber 31. The diameter of the Teflon tube may be consistent with the diameter of the first pipeline 21, the second pipeline 22, or the bypass pipeline 23, and the length of the Teflon tube may be set according to the gas content in the liquid. The negative pressure condition refers to a pressure range in the sealed chamber 31 that allows the gas in the liquid in the pipeline 20 to permeate out.

Referring to FIG 2, in some embodiments, the degassing device 30 includes a negative pressure device 32 and an exhaust pipeline 33. One end of the exhaust pipeline 33 is in communication with the sealed chamber 31, and the negative pressure device 32 is arranged on the exhaust pipeline 33 and configured to generate a negative pressure in the sealed chamber 31.

In this way, the negative pressure device 32 creates a negative pressure in the sealed chamber 31, so that the gas in the liquid in the pipeline 20 can be discharged after passing through the sealed chamber 31.

Specifically, the negative pressure device 32 may be a vacuum pump, including but not limited to a water ring pump, a reciprocating pump, a slide valve pump, a rotary vane pump, a Roots pump, and a diffusion pump. The negative pressure device 32 sucks air out of the sealed chamber 31 to generate a negative pressure within the sealed chamber 31.

The exhaust pipeline 33 can deliver gas in the sealed chamber 31 to the negative pressure device 32, and the negative pressure device 32 then expels the gas to the atmosphere, thereby creating a negative pressure condition within the sealed chamber 31. The gas in the sealed chamber 31 may be the gas removed from the first pipeline 21, the second pipeline 22, or the bypass pipeline 23. Referring to FIG 2, in some embodiments, the degassing device 30 includes a detector 34 located between the negative pressure device 32 and the sealed chamber 31. The detector 34 is in communication with the exhaust pipeline 33, and the detector 34 is configured to detect the air pressure in the sealed chamber 31 through the exhaust pipeline 33.

Specifically, the detector 34 may be a pressure sensor, which is a device that utilizes principles such as piezoresistive, capacitive, piezoelectric, or crystal resonance to convert pressure into an electrical signal. The detector 34 may be in communication with the exhaust pipeline 33 via a three-way connector 35.

By detecting the air pressure in the sealed chamber 31 with the detector 34, it can be determined whether the air pressure in the sealed chamber 31 has reached the negative pressure condition. When the air pressure in the sealed chamber 31 has not reached the negative pressure condition, the degassing device 30 is started. When the air pressure in the sealed chamber 31 has reached the negative pressure condition, the negative pressure device 32 is turned off. By adjusting the operating state of the negative pressure device 32, the sealed chamber 31 is maintained in a negative pressure state, thereby improving the degassing efficiency of the degassing device 30. Meanwhile, the on and off time of the negative pressure device 32 can be controlled. In this way, the usage frequency of the negative pressure device 32 can be effectively reduced, thereby improving the service life of the negative pressure device 32.

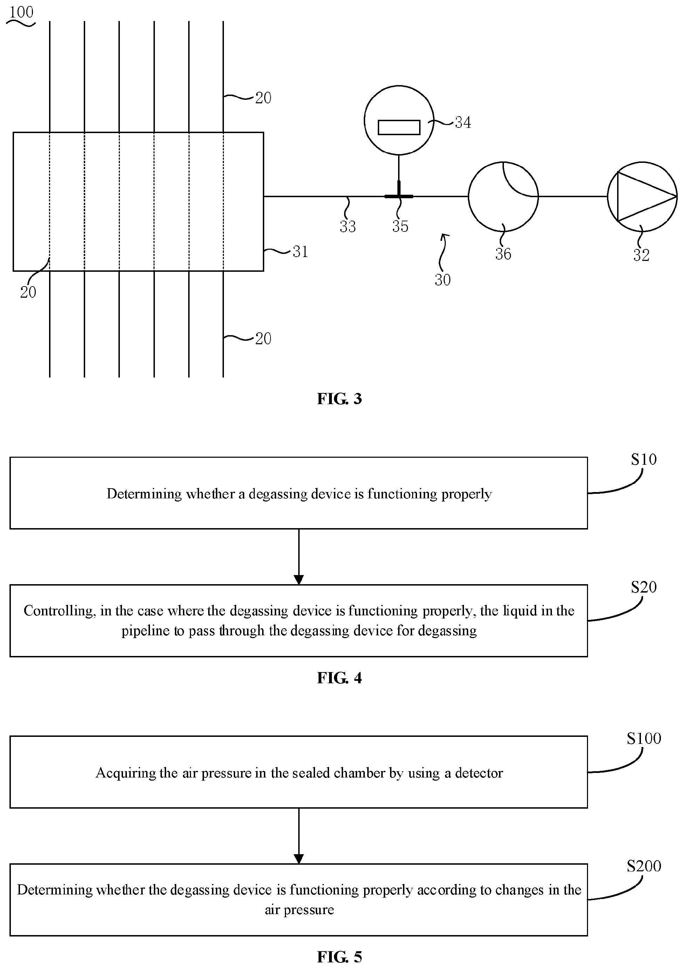

Referring to FIG 3, in some embodiments, the degassing device 30 includes a multi-way valve 36 arranged on the exhaust pipeline 33 and located between the detector 34 and the negative pressure device 32. The multi-way valve 36 is capable of enabling the negative pressure device 32 to communicate with either the sealed chamber 31 or the atmosphere.

Specifically, the multi-way valve 36 may be a three-way valve, with the three ports of the three-way valve respectively in communication with the negative pressure device 32, the sealed chamber 31, and the atmosphere. In one embodiment, a negative pressure device 32 without a check function may be selected. When the negative pressure device 32 is operating, the multi-way valve 36 communicates the negative pressure device 32 with the sealed chamber 31, allowing the negative pressure device 32 to suck out the air within the sealed chamber 31 to create a negative pressure. When the negative pressure device 32 stops operating, the multi-way valve 36 communicates the negative pressure device 32 with the atmosphere, which can reduce the risk that, when the negative pressure device 32 starts, the high air pressure in the sealed chamber 31 will prevent or hinder the start-up of the negative pressure device 32 and consequently shorten the service life of the negative pressure device 32.

In another embodiment, a negative pressure device 32 with a one-way check function may be selected, which can reduce the use of solenoid valves, lower the cost of the fluidic system 100, and avoid failures of solenoid valves, thereby improving the reliability of the degassing device 30. Referring to FIG 4, the present application provides a method for controlling degassing of liquid in the fluidic system 100. The method includes:
S10, determining whether the degassing device 30 is functioning properly; and
S20, controlling, in the case where the degassing device 30 is functioning properly, the liquid in the pipeline 20 to pass through the degassing device 30 for degassing.

Specifically, the determination of whether the degassing device 30 is functioning properly may be performed before activating the fluidic system 100 or during the operation of the fluidic system 100. The degassing device 30 is considered to be functioning properly if all components in the degassing device 30 are operating properly, such as the sealed chamber 31 having proper hermeticity, the exhaust pipeline 33 having proper hermeticity, the negative pressure device 32 functioning properly, and the detector 34 functioning properly. The degassing device 30 is considered to be malfunctioning if one or more components in the degassing device 30 are abnormal, such as abnormal hermeticity of the sealed chamber 31, abnormal hermeticity of the exhaust pipeline 33, abnormal operation of the negative pressure device 32, or abnormal operation of the detector 34.

In this way, by determining whether the degassing device 30 is functioning properly, it is determined whether or not to activate the degassing device 30.

Referring to FIG 5, in some embodiments, determining whether the degassing device 30 is functioning properly (step S10) includes:
S100, acquiring the air pressure in the sealed chamber 31 by using the detector 34; and
S200, determining whether the degassing device 30 is functioning properly according to changes in the air pressure.

Specifically, before acquiring the air pressure in the sealed chamber 31 by using the detector 34, the detector 34 may first be checked to ensure that the detector 34 is functioning properly.

The change in air pressure may refer to the amount of air pressure change within a certain period of time, or the time difference required for the air pressure to change by a certain amount. The determination of whether the degassing device 30 is functioning properly may be performed while the negative pressure device 32 is operating. Alternatively, the determination of whether the degassing device 30 is functioning properly may be performed when the negative pressure device 32 stops operating. In this way, whether the degassing device 30 is functioning properly can be monitored throughout the entire operation process of the fluidic system 100, so as to sufficiently remove gas from the liquid in the pipeline 20.

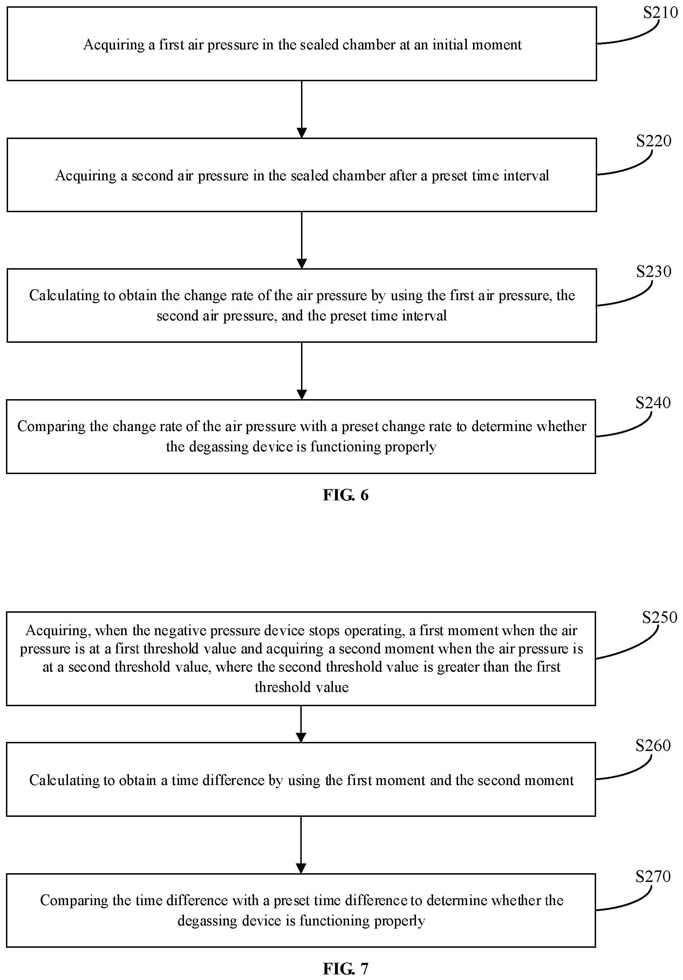

Referring to FIG 6, in some embodiments, determining whether the degassing device 30 is functioning properly according to the changes in the air pressure (step S200) includes:
S210, acquiring a first air pressure in the sealed chamber 31 at an initial moment;
S220, acquiring a second air pressure in the sealed chamber 31 after a preset time interval;
S230, calculating to obtain the change rate of the air pressure by using the first air pressure, the second air pressure, and the preset time interval; and
S240, comparing the change rate of the air pressure with a preset change rate to determine whether the degassing device 30 is functioning properly.

Specifically, the initial moment may be the moment when the negative pressure device 32 starts operating, or the moment when the negative pressure device 32 stops operating. The preset time interval may be the duration of operation of the negative pressure device 32, or the duration of stoppage of the negative pressure device 32.

The air pressure change rate may be the ratio of the difference between the second air pressure and the first air pressure to the preset time interval, and the preset change rate is the change rate of the air pressure in the sealed chamber 31 within the preset time interval when the degassing device 30 is functioning properly. The preset change rate may be a range. When the air pressure change rate falls within the range, it is determined that the degassing device 30 is functioning properly; when the air pressure change rate falls outside the range, it is determined that the degassing device 30 is malfunctioning.

In this way, by calculating the air pressure change rate within the preset time interval and comparing the air pressure change rate with the preset change rate, it is possible to objectively and accurately determine whether the degassing device 30 is functioning properly.

In an embodiment, the air pressure difference over the preset time interval may be calculated using the first air pressure and the second air pressure, and then the air pressure difference over the preset time interval is compared with the preset air pressure difference for the preset time interval to determine whether the degassing device 30 is functioning properly.

In some embodiments, comparing the air pressure change rate with the preset change rate to determine whether the degassing device 30 is functioning properly includes:
When the negative pressure device 32 is operating, if the air pressure change rate is greater than or equal to the preset change rate, it is determined that the degassing device 30 is functioning properly; if the air pressure change rate is less than the preset change rate, it is determined that the degassing device 30 is malfunctioning.

Specifically, when the negative pressure device 32 is operating, if the air pressure change rate is greater than or equal to the preset change rate, it indicates that the air pressure in the sealed chamber 31 reaches the negative pressure condition within the preset time interval, and that the hermeticity of the sealed chamber 31, the hermeticity of the exhaust pipeline 33, and the negative pressure device 32 are all proper; therefore, it can be determined that the degassing device 30 is functioning properly.

If the air pressure change rate is less than the preset change rate, it indicates that the air pressure in the sealed chamber 31 does not reach the negative pressure condition within the preset time interval, and that the hermeticity of the sealed chamber 31, the hermeticity of the exhaust pipeline 33, or the negative pressure device 32 is abnormal; therefore, it can be determined that the degassing device 30 is malfunctioning.

In some embodiments, comparing the air pressure change rate with the preset change rate to determine whether the degassing device 30 is functioning properly includes:
When the negative pressure device 32 stops operating, if the air pressure change rate is less than or equal to the preset change rate, it is determined that the degassing device 30 is functioning properly; if the air pressure change rate is greater than the preset change rate, it is determined that the degassing device 30 is malfunctioning.

Specifically, when the negative pressure device 32 stops operating, if the air pressure change rate is less than or equal to the preset change rate, it indicates that the hermeticity of the sealed chamber 31 and the hermeticity of the exhaust pipeline 33 are proper; therefore, it can be determined that the degassing device 30 is functioning properly.

If the air pressure change rate is greater than the preset change rate, it indicates that the hermeticity of the sealed chamber 31 or the hermeticity of the exhaust pipeline 33 is abnormal; therefore, it can be determined that the degassing device 30 is malfunctioning.

Referring to FIG 7, in some embodiments, determining whether the degassing device 30 is functioning properly according to the changes in the air pressure (step S200) includes:
S250, acquiring, when the negative pressure device 32 stops operating, a first moment when the air pressure is at a first threshold value and acquiring a second moment when the air pressure is at a second threshold value, where the second threshold value is greater than the first threshold value;
S260, calculating to obtain a time difference by using the first moment and the second moment; and
S270, comparing the time difference with a preset time difference to determine whether the degassing device 30 is functioning properly.

Specifically, the first threshold value may be the lower limit value at which the air pressure in the sealed chamber 31 reaches the negative pressure condition, and the second threshold value may be the upper limit value at which the air pressure in the sealed chamber 31 reaches the negative pressure condition. Both the upper limit value and the lower limit value are negative values.

The time difference may be the difference between the first moment and the second moment, and the preset time difference is the duration required for the air pressure in the sealed chamber 31 to change from the first threshold value to the second threshold value when the degassing device 30 is functioning properly. The preset time difference may be a range. When the time difference for the air pressure to change from the first threshold value to the second threshold value falls within the range, it is determined that the degassing device 30 is functioning properly; when the time difference falls outside the range, it is determined that the degassing device 30 is malfunctioning. In this way, by comparing the time difference for the air pressure to change from the first threshold value to the second threshold value with the preset time difference, it is possible to objectively and accurately determine whether the degassing device 30 is functioning properly. Moreover, it can simplify the calculation steps, making the calculation process simple and quick, thereby improving the determination efficiency.

In some embodiments, comparing the time difference for the air pressure to change from the first threshold value to the second threshold value with the preset time difference to determine whether the degassing device 30 is functioning properly includes:
If the time difference for the air pressure to change from the first threshold value to the second threshold value is greater than or equal to the preset time difference, it is determined that the degassing device 30 is functioning properly; if the time difference for the air pressure to change from the first threshold value to the second threshold value is less than the preset time difference, it is determined that the degassing device 30 is malfunctioning.

Specifically, when the negative pressure device 32 stops operating, if the time difference for the air pressure to change from the first threshold value to the second threshold value is greater than or equal to the preset time difference, it indicates that the air pressure in the sealed chamber 31 remains under the negative pressure condition within the preset time difference, and the hermeticity of the sealed chamber 31 and the hermeticity of the exhaust pipeline 33 are normal; therefore, it can be determined that the degassing device 30 is functioning properly.

If the time difference for the air pressure to change from the first threshold value to the second threshold value is less than the preset time difference, it indicates that the air pressure in the sealed chamber 31 does not reach the negative pressure condition within the preset time difference, and that the hermeticity of the sealed chamber 31 or the hermeticity of the exhaust pipeline 33 is abnormal; therefore, it can be determined that the degassing device 30 is malfunctioning.

In some embodiments, determining whether the degassing device 30 is functioning properly according to the changes in the air pressure includes:
recording, when the negative pressure device 32 stops operating, the air pressure as a first threshold value;
starting, when the air pressure is at a second threshold value, the negative pressure device 32; and
determining, if the air pressure is greater than the first threshold value within the preset time, that the degassing device 30 is malfunctioning; or determining, if the air pressure is less than or equal to the first threshold value within the preset time, that the degassing device 30 is functioning properly, where the second threshold value is greater than the first threshold value.

Specifically, when the negative pressure device 32 is operating, if the air pressure is greater than the first threshold value within the preset time, it indicates that the air pressure in the sealed chamber 31 does not reach the negative pressure condition within the preset time difference, and that the hermeticity of the sealed chamber 31, the hermeticity of the exhaust pipeline 33, or the negative pressure device 32 is abnormal; therefore, it can be determined that the degassing device 30 is malfunctioning.

If the air pressure is less than or equal to the first threshold value within the preset time, it indicates that the air pressure in the sealed chamber 31 reaches the negative pressure condition within the preset time difference, and that the hermeticity of the sealed chamber 31, the hermeticity of the exhaust pipeline 33, and the negative pressure device 32 are all normal; therefore, it can be determined that the degassing device 30 is functioning properly.

In some embodiments, determining whether the degassing device 30 is functioning properly according to the changes in the air pressure includes:
recording, when the negative pressure device 32 stops operating, the air pressure as a first threshold value;
determining, at the moment of the preset time interval, whether the air pressure in the sealed chamber 31 is greater than the first threshold value;
acquiring, if the air pressure in the sealed chamber 31 is greater than the first threshold value, the change rate of the air pressure; and
determining, if the change rate of the air pressure is greater than the preset change rate, that the degassing device 30 is malfunctioning; otherwise, determining that the degassing device 30 is functioning properly.

Specifically, when the negative pressure device 32 stops operating, the air pressure is the lower limit value at which the sealed chamber 31 reaches the negative pressure condition, and as the gas in the liquid in the pipeline 20 enters the sealed chamber 31 from the pipeline 20 under the action of negative pressure, the air pressure in the sealed chamber 31 rises.

If the change rate of the air pressure is greater than the preset change rate, it indicates that gas from the external environment enters the sealed chamber 31, and further indicates that the hermeticity of the sealed chamber 31 or the hermeticity of the exhaust pipeline 33 is abnormal; therefore, it can be determined that the degassing device 30 is malfunctioning.

If the change rate of the air pressure is less than or equal to the preset change rate, it indicates that the hermeticity of the sealed chamber 31 and the hermeticity of the exhaust pipeline 33 are normal; therefore, it can be determined that the degassing device 30 is functioning properly.

In some embodiments, the method further includes:
starting, if the degassing device 30 is functioning properly, the negative pressure device 32; or
not starting, if the degassing device 30 is malfunctioning, the negative pressure device 32.

Specifically, before activating the fluidic system 100, the negative pressure device 32 can be started to determine whether the degassing device 30 is functioning properly. If the degassing device 30 is functioning properly, the negative pressure device 32 is started during the operation of the fluidic system 100. If the degassing device 30 is malfunctioning, the negative pressure device 32 is not started during the operation of the fluidic system 100.

During the operation of the fluidic system 100, whether the degassing device 30 is functioning properly can be determined at regular intervals. If the degassing device 30 is functioning properly, when the air pressure in the sealed chamber 31 does not reach the negative pressure condition, the degassing device 30 is started. When the air pressure in the sealed chamber 31 reaches the negative pressure condition, the negative pressure device 32 is turned off. If the degassing device 30 is malfunctioning, the negative pressure device 32 is not started during the subsequent operation of the fluidic system 100.

In one embodiment, the detector 34 can be connected to a computer device, and the computer determines whether the degassing device 30 is functioning properly based on changes in the air pressure. When the degassing device 30 is malfunctioning, the computer can issue an alert and turn off the negative pressure device 32 that is operating.

In this way, starting the degassing device 30 when the degassing device 30 is functioning properly can effectively remove gas from the liquid in the pipeline 20. Turning off the degassing device 30 in the case where the degassing device 30 is malfunctioning can reduce the waste of electric power resources and the consumption of the service life of the negative pressure device 32.

In some embodiments, the degassing device 30 includes a first degassing device 37, and the first pipeline 21 is connected to the first degassing device 37.

Controlling, in the case where the degassing device 30 is functioning properly, the liquid in the pipeline 20 to pass through the degassing device 30 for degassing includes:
controlling the liquid in the first pipeline 21 to pass through the first degassing device 37 for degassing, and causing the degassed liquid to enter the reaction device 10.

Specifically, a negative pressure condition is formed within the sealed chamber 31 of the first degassing device 37. The gas in the liquid in the first pipeline 21 flows out from the first pipeline 21 into the sealed chamber 31 as it passes through the sealed chamber 31. The gas in the sealed chamber 31 is then discharged to the atmosphere through the exhaust pipeline 33. The liquid in the first pipeline 21 passing through the sealed chamber 31 enters the reaction device 10 from the liquid inlet 11.

In this way, the gas in the liquid in the first pipeline 21 can be removed, thereby reducing or avoiding the formation of bubbles in the reaction device 10.

In some embodiments, the degassing device 30 includes a second degassing device 38, and the second pipeline 22 is connected to the second degassing device 38.

Controlling, in the case where the degassing device 30 is functioning properly, the liquid in the pipeline 20 to pass through the degassing device 30 for degassing includes:
controlling the liquid that flows out of the reaction device 10 and enters the second pipeline 22 to pass through the second degassing device 38 for degassing.

Specifically, the selection of components in the second degassing device 38 may be the same as or different from that in the first degassing device 37. A negative pressure condition is formed within the sealed chamber 31 of the second degassing device 38. The gas in the liquid in the second pipeline 22 flows out from the second pipeline 22 into the sealed chamber 31 as it passes through the sealed chamber 31. The gas in the sealed chamber 31 is then discharged to the atmosphere through the exhaust pipeline 33. The liquid in the second pipeline 22 passing through the sealed chamber 31 can be stored in the second pipeline 22 or the bypass pipeline 23 for reuse.

In this way, the gas in the liquid in the second pipeline 22 can be removed, thereby reducing or avoiding the formation of bubbles in the reaction device 10 during the reuse of liquid.

In some embodiments, the degassing device 30 includes a third degassing device 39, and the bypass pipeline 23 is connected to the third degassing device 39.

Controlling, in the case where the degassing device 30 is functioning properly, the liquid in the pipeline 20 to pass through the degassing device 30 for degassing includes:
controlling the liquid that flows out of the reaction device 10 to enter the bypass pipeline 23, and causing the liquid in the bypass pipeline 23 to enter the third degassing device 39 for degassing.

Specifically, the selection of components in the third degassing device 39 can be the same as or different from that in the first degassing device 37 or the second degassing device 38. The liquid that has been degassed by the second degassing device 38 can enter the bypass pipeline 23. A negative pressure condition is formed within the sealed chamber 31 of the third degassing device 39. The gas in the liquid in the bypass pipeline 23 flows out from the bypass pipeline 23 into the sealed chamber 31 as it passes through the sealed chamber 31. The gas in the sealed chamber 31 is then discharged to the atmosphere through the exhaust pipeline 33. The liquid in the bypass pipeline 23 passing through the sealed chamber 31 can be stored in the bypass pipeline 23 for reuse.

In this way, the gas in the liquid in the bypass pipeline 23 can be removed, thereby reducing or avoiding the formation of bubbles in the reaction device 10 during the reuse of liquid.

In some embodiments, the method further includes:
causing the degassed liquid in the bypass pipeline 23 to enter the reaction device 10.

Specifically, the liquid that has been degassed by the third degassing device 39 enters the first pipeline 21 and then enters the reaction device 10 from the liquid inlet 11. In this way, liquid reuse can be achieved, thereby reducing costs.

The embodiments of the present application provide a sequencing system, which includes the fluidic system 100 described above.

The embodiments of the present application provide a computer storage medium. A computer program, when run by a processor, causes the processor to implement the method for controlling degassing of liquid in the fluidic system 100 according to any one of the above embodiments. Specifically, in one embodiment, the processor may be a central processing unit (CPU). The processor may also be other general-purpose processors, digital signal processors (DSP), application specific integrated circuits (ASIC), field-programmable gate arrays (FPGA), or other chips such as programmable logic devices, discrete gates, transistor logic devices, discrete hardware components, or a combination thereof.

The computer program can be stored in a memory. The memory, as a non-transitory computer-readable storage medium, can be configured to store non-transitory software programs, non-transitory computer-executable programs, and modules, such as program instructions/modules corresponding to the methods in the aforementioned method embodiments. The processor, by executing non-transitory software programs, instructions, and modules stored in the memory, performs various functional applications and data processing of the processor, thereby implementing the control method in the aforementioned method embodiments.

The storage medium may include, but is not limited to, various media capable of storing computer programs, such as a U disk, a read-only memory (ROM), a random access memory (RAM), a portable hard disk, a magnetic disk, or an optical disk.

In the description of the specification, references to the terms such as "one embodiment", "some embodiments", "schematic embodiments", "examples", "specific examples", or "some examples" mean that the specific feature, structure, material, or characteristic described in connection with the embodiment or example is included in at least one embodiment or example of the present disclosure. In this specification, the schematic description of the aforementioned terms does not necessarily refer to the same embodiment or example. Moreover, the particular features, structures, materials, or characteristics described may be combined in any embodiment or example in any appropriate manner.

Although the embodiments of the present disclosure have been illustrated and described, it can be understood by those of ordinary skill in the art that various changes, modifications, replacements, and variations can be made to these embodiments without departing from the principle and purpose of the present disclosure, and the scope of the present disclosure is defined by the claims and equivalents therefore.

## Claims

1. A fluidic system, comprising:
a reaction device, configured to provide a site for carrying out biochemical reactions;
a pipeline in communication with the reaction device and configured to deliver liquid to the reaction device and/or receive liquid flowing out from the reaction device; and
a degassing device connected to the pipeline and configured to remove gas from the liquid in the pipeline.

2. The fluidic system according to claim 1, wherein
the reaction device comprises a liquid inlet and a liquid outlet; and
the pipeline comprises a first pipeline and a second pipeline, wherein the first pipeline is in communication with the liquid inlet, and the second pipeline is in communication with the liquid outlet; the first pipeline and/or the second pipeline is connected to the degassing device;
optionally, the reaction device comprises a liquid inlet and a liquid outlet; and the pipeline comprises a bypass pipeline, wherein one end of the bypass pipeline is in communication with the liquid inlet, and the other end is in communication with the liquid outlet; the bypass pipeline is connected to the degassing device.

3. The fluidic system according to claim 1 or 2, wherein
the degassing device comprises a sealed chamber, the pipeline passes through the sealed chamber, and a portion of the pipeline located within the sealed chamber is capable of, when the sealed chamber is under a negative pressure condition, allowing the gas in the liquid in the pipeline to permeate out, so that the liquid in the pipeline is degassed after passing through the sealed chamber.

4. The fluidic system according to claim 3, wherein
the degassing device comprises a negative pressure device and an exhaust pipeline, wherein one end of the exhaust pipeline is in communication with the sealed chamber, and the negative pressure device is arranged on the exhaust pipeline and configured to generate a negative pressure in the sealed chamber.

5. The fluidic system according to claim 4, wherein
the degassing device comprises a detector located between the negative pressure device and the sealed chamber, wherein the detector is in communication with the exhaust pipeline and configured to detect an air pressure in the sealed chamber through the exhaust pipeline;
optionally, the degassing device comprises a multi-way valve arranged on the exhaust pipeline and located between the detector and the negative pressure device, wherein the multi-way valve is capable of enabling the negative pressure device to communicate with either the sealed chamber or the atmosphere.

6. A method for controlling degassing of liquid in a fluidic system, wherein the fluidic system comprises:
a reaction device, configured to provide a site for carrying out biochemical reactions;
a pipeline in communication with the reaction device and configured to deliver liquid to the reaction device and/or receive liquid flowing out from the reaction device; and
a degassing device connected to the pipeline and configured to remove gas from the liquid in the pipeline; and wherein
the method comprises:
determining whether the degassing device is functioning properly; and
controlling, in a case where the degassing device is functioning properly, the liquid in the pipeline to pass through the degassing device for degassing.

7. The method according to claim 6, wherein
the degassing device comprises a sealed chamber, the pipeline passes through the sealed chamber, and a portion of the pipeline located within the sealed chamber is capable of, when the sealed chamber is under a negative pressure condition, allowing the gas in the liquid in the pipeline to permeate out, so that the liquid in the pipeline is degassed after passing through the sealed chamber;

8. The method according to claim 7, wherein
the degassing device comprises a negative pressure device and an exhaust pipeline, wherein one end of the exhaust pipeline is in communication with the sealed chamber, and the negative pressure device is arranged on the exhaust pipeline and configured to generate a negative pressure in the sealed chamber;

9. The method according to claim 8, wherein
the degassing device comprises a detector located between the negative pressure device and the sealed chamber, wherein the detector is in communication with the exhaust pipeline and configured to detect an air pressure in the sealed chamber through the exhaust pipeline;
determining whether the degassing device is functioning properly comprises:
acquiring the air pressure in the sealed chamber by using the detector; and
determining whether the degassing device is functioning properly according to changes in the air pressure.

10. The method according to claim 9, wherein
determining whether the degassing device is functioning properly according to the changes in the air pressure comprises:
acquiring a first air pressure in the sealed chamber at an initial moment;
acquiring a second air pressure in the sealed chamber after a preset time interval;
calculating to obtain a change rate of the air pressure by using the first air pressure, the second air pressure, and the preset time interval; and
comparing the change rate of the air pressure with a preset change rate to determine whether the degassing device is functioning properly;
optionally, comparing the change rate of the air pressure with the preset change rate to determine whether the degassing device is functioning properly comprises:
when the negative pressure device is operating, determining, if the change rate of the air pressure is greater than or equal to the preset change rate, that the degassing device is functioning properly; or determining, if the change rate of the air pressure is less than the preset change rate, that the degassing device is malfunctioning;
optionally, wherein comparing the change rate of the air pressure with the preset change rate to determine whether the degassing device is functioning properly comprises:
when the negative pressure device stops operating, determining, if the change rate of the air pressure is less than or equal to the preset change rate, that the degassing device is functioning properly; or determining, if the change rate of the air pressure is greater than the preset change rate, that the degassing device is malfunctioning.

11. The method according to claim 9, wherein determining whether the degassing device is functioning properly according to the changes in the air pressure comprises:
acquiring, when the negative pressure device stops operating, a first moment when the air pressure is at a first threshold value and acquiring a second moment when the air pressure is at a second threshold value, wherein the second threshold value is greater than the first threshold value;
calculating to obtain a time difference by using the first moment and the second moment; and
comparing the time difference with a preset time difference to determine whether the degassing device is functioning properly;
optionally, wherein comparing the time difference with the preset time difference to determine whether the degassing device is functioning properly comprises:
determining, if the time difference is greater than or equal to the preset time difference, that the degassing device is functioning properly; or determining, if the time difference is less than the preset time difference, that the degassing device is malfunctioning.

12. The method according to claim 9, wherein determining whether the degassing device is functioning properly according to the changes in the air pressure comprises:
recording, when the negative pressure device stops operating, the air pressure as a first threshold value;
starting, when the air pressure is at a second threshold value, the negative pressure device; and
determining, if the air pressure is greater than the first threshold value within a preset time, that the degassing device is malfunctioning; or determining, if the air pressure is less than or equal to the first threshold value within the preset time, that the degassing device is functioning properly, wherein the second threshold value is greater than the first threshold value.

13. The method according to claim 9, wherein determining whether the degassing device is functioning properly according to the changes in the air pressure comprises:
recording, when the negative pressure device stops operating, the air pressure as a first threshold value;
determining, at a first moment, whether the air pressure in the sealed chamber is greater than the first threshold value;
acquiring, if the air pressure in the sealed chamber is greater than the first threshold value, a change rate of the air pressure; and
determining, if the change rate is greater than a preset change rate, that the degassing device is malfunctioning; otherwise, determining that the degassing device is functioning properly.

14. The method according to any one of claims 9 to 13, further comprising:
starting, if the degassing device is functioning properly, the negative pressure device; or
not starting, if the degassing device is malfunctioning, the negative pressure device.

15. The method according to any one of claims 8 to 19, wherein
the reaction device comprises a liquid inlet and a liquid outlet; and
the pipeline comprises a first pipeline and a second pipeline, wherein the first pipeline is in communication with the liquid inlet, and the second pipeline is in communication with the liquid outlet; the degassing device comprises a first degassing device, and the first pipeline is connected to the first degassing device; and wherein
controlling, in the case where the degassing device is functioning properly, the liquid in the pipeline to pass through the degassing device for degassing comprises:
controlling the liquid in the first pipeline to pass through the first degassing device for degassing, and causing the degassed liquid to enter the reaction device;
optionally, the degassing device comprises a second degassing device, and the second pipeline is connected to the second degassing device; and wherein
controlling, in the case where the degassing device is functioning properly, the liquid in the pipeline to pass through the degassing device for degassing comprises:
controlling the liquid that flows out of the reaction device and enters the second pipeline to pass through the second degassing device for degassing;
optionally, the degassing device comprises a third degassing device; and
the pipeline comprises a bypass pipeline, wherein one end of the bypass pipeline is in communication with the liquid inlet, and the other end is in communication with the liquid outlet;
the bypass pipeline is connected to the third degassing device; and wherein
controlling, in the case where the degassing device is functioning properly, the liquid in the pipeline to pass through the degassing device for degassing comprises:
controlling the liquid that flows out of the reaction device to enter the bypass pipeline, and causing the liquid in the bypass pipeline to enter the third degassing device for degassing;
optionally, further comprising:
causing the degassed liquid in the bypass pipeline to enter the reaction device.
